# EUROPEAN PATENT APPLICATION

(11) **EP 4 309 704 A1**
(43) Date of publication of application: **24.01.2024**
(21) Application number: 22186290.7
(22) Date of filing: 21.07.2022
(51) Int. Cl.: A61M 5/20, A61M 5/315

(54) **AN INJECTION DEVICE COMPRISING A CLOSURE CAP THAT CAN BE REMOVED TOGETHER WITH AN ELECTRONIC MODULE**

(71) Applicant: Ypsomed AG, 3401 Burgdorf (CH)
(72) Inventor: Kühni, Florian, 3007 Bern (CH); Bosshard, Simon Martin, 3324 Hindelbank (CH); Zumstein, Dominik, 3014 Bern (CH); Schrul, Christian, 3400 Burgdorf (CH); Etter, Leoni, 3011 Bern (CH)
(74) Representative: Meier Obertüfer, Jürg

(57) **Abstract**

An injection device for expelling fluid medicament comprising a housing (2) and a closure cap (17) closing the housing (2), The closure cap (17) being connected to the housing by a first releasable connection, and a printed circuit board (16c) comprising electronic elements, the printed circuit board (16c) being located in the closure cap (17). A first connector (17g) is located on the closure cap (17) and a second connector (16d) is located on the printed circuit board (16c). A second connection is configured to be established between the closure cap (17) and the printed circuit board (16c) by engaging the first and second connection means (17g, 16d) through a relative movement between the closure cap (17) and the housing (2) as the medicament delivery device is fully assembled.

## Description

### TECHNICAL FIELD

The current invention relates to an injection device comprising a releasable connection for a closure cap closing the injection device. The closure cap can be removed together with an electronic module.

### BACKGROUND OF THE INVENTION

Medicament delivery devices such as, for example injections pens, auto injectors, autopens, infusion pumps, patch injectors, bolus injectors or patch pumps use a drive mechanism for expelling medicament from the device. The drive mechanism comprises mechanical components for advancing, for example, a piston rod forwarding a bung in a reservoir or to drive a pumping mechanism. The medicament delivery devices may be disposable or reusable devices. For disposable delivery devices, the mechanical components are disposed after use and may be used for recycling specific components or the raw materials if the device is shredded. Alternatively, the device is combusted or ends at a waste depository.

There is a need to include additional features to those mechanical devices for example by adding additional modules to the mechanical device. Preferably such an additional module is an electronic module and examples for those electronic modules may be a connectivity module with a transmitter and/or receiver to allow communication with other devices, a sensing module to allow for sensing patient parameters such as blood glucose values, a location module indicating geographical locations, a timer or calendar module, a communication or training module including a loudspeaker, or a sensing module to detect impact. Those additional electronic modules may be added as an add-on device or supplementary device to the existing medicament delivery device. The add-on may be a separate add on that the end user attaches to the delivery device or the add-on is integrated into the (mechanical) delivery device by the device manufacturer, a so called integrated add-on.

The separate add-on or integrated add-on device itself may be disposable or reusable. For a reusable add-on device combined with a disposable delivery device, the add-on must be removed from the disposable delivery device for re-use of the add-on. For a disposable add-on it may be beneficial to separate the add-on from the mechanical components of the delivery device for separate waste treatment as the add-on devices include electronic components and batteries requiring a different treatment compared to the mechanical components.

The electronic components may include a printed circuit board (PCB) providing mechanical stability and a platform for integrated circuits, electrical connections, memory units, processors and the like. The electronic components are powered by a battery that may be fixated to the PCB as well. The PCB including all electronic components needs to be reliable, for example to guarantee the transmittance of data to an external device during or after use. It is desired that the PCB comprising delicate components and electrical connections is not exposed to excessive mechanical forces and protected from the ambient. Long term bending forces may, for example, lead to delamination of electrical connections from the PCB or disconnection between parts. In addition it is desired to enclose the PCB in a housing part or closure cap that may be attached to the housing enclosing the mechanical components or to an end surface or end cap of the housing. The closure cap may be designed to minimize the mechanical forces acting on the PCB before use for example by using flexible closure caps. The closure cap protects the PCB from the ambient during shelf-life of the product. The closure cap furthermore prevents that a user can directly see, touch or modify the components located on the PCB, and, additionally, protects the PCB from contamination before use. After use, the PCB may be removed from the device for recycling purposes requiring removal of the closure cap. The PCB will be exposed to the user or may even be detached from the device or closure cap leading to discomfort to the user as the user is not expected to see the PCB and all its components.

There is a need to reduce waste and to facilitate re-use of components or materials or to recycle materials to reduce the amount of raw material or energy used throughout the lifecycle of the product. Thus there is a need to improve the medicament delivery devices including add-on features such that they are designed for recycling.

In US7008399 an injection pen is disclosed having a printed circuit board, a battery, a LCD located behind a window in the dose setting knob. The pen is fully integrated and there are no precautions taken to separate the electronic components from the mechanical components.

WO2018041798 discloses a reusable supplementary device that can be releasably connected to the housing of an injection device. The mechanical connection comprises snap fit connectors comprising hooks on flexible latching arms extending axially from the supplementary device engaging recesses in the housing. The mechanical connection is used for forming an optical coupling between the injection device and the supplementary device.

In EP 21187112.4 an autoinjector is disclosed with a module housing closing an electronic module that is releasable attached to a housing comprising the injection mechanism. The module housing can be separated from the housing after injection for recycling purposes.

It is an object of the present invention to improve the injection or infusion devices for recycling purposes by providing closure caps that enable removal of the PCB after medicament delivery. This objective is solved by the independent claim and specific variants are disclosed in the dependent claims. Furthermore a method facilitating the recycling of a PCB is presented.

### DEFINITIONS

The term "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The distal end or distal direction is defined by the direction of the hollow needle configured to penetrate the skin of the patient. For an injection device such as an injection pen this may be the injection needle and the end of the pen holding the needle or being configured to hold the needle is the distal end. For an infusion device the distal end and the distal direction is towards the needle configured to penetrate the skin of the patient, which may be along the axis of the device or tilted or perpendicular to the axis of the device. The distal direction in an infusion device represents the direction in which the medicament flows towards the insertion needle. The proximal direction or end is opposite to the distal direction or end.

The PCB in the context of this invention may relate to the board itself or to the board including all electronic components. Optionally, the battery is part of the PCB as well.

In the claims, the word "comprising" does not exclude other elements or steps, and the indefinite article "a" or "an" does not exclude a plurality. For example "a connector" or "connection means" does not exclude the fact that there may be two connectors that functionally or structurally fulfill the purpose of "a connector". The mere fact that certain elements or steps are recited in distinct claims shall not preclude the existence of further meaningful combinations of these elements or steps.

### GENERAL DESCRIPTION OF THE INVENTION

In the current invention a delivery device for expelling fluid medicament is disclosed and the delivery device comprises a housing and a closure cap for the housing which may be connected to the housing by a first releasable connection. An electronic module comprising a printed circuit board comprising electronic components is preferably located in or surrounded by the closure cap. Examples of the electronic module may include one or more of a connectivity module with a transmitter and/or receiver to allow communication with other devices, a sensing module to allow for sensing patient parameters such as blood glucose values, a location module indicating geographical locations, a timer or calendar module, a communication or training module including a loudspeaker, or a sensing module to detect impact. The electronic module may be part of the user interface and may include an electronic display or LED indicators which may provide guidance to the user when using the injection device. The guidance may be, for example the holding time for holding an injection device in contact with the patient's skin after medicament delivery has been completed. The electronic modules preferably use a printed circuit board as a platform holding the electronic components. A first connection means (or first connector or first fixator) may be located on the closure cap and a second connection means (or second connector or second fixator) may be located on the printed circuit board (PCB). A second connection is configured to be established between the closure cap and the printed circuit board by engaging the first and second connection means through a relative movement between the closure cap and the housing, preferably between the closure cap and the PCB in a fully assembled state of the medicament delivery device. The second connection may also be a releasable connection.

The first releasable connection is preferably released by the user which may be a patient or a health care professional. Preferably, the second connection between the closure cap and the printed circuit board is established before releasing the first releasable connection between the closure cap and the housing or a housing part such as, for example, an end cap for the housing. This option has the advantage that the PCB is fixated to the closure cap when the closure cap is removed from the device. Optionally, the second releasable connection is formed simultaneously with releasing the first releasable connection. As another option, the second releasable connection between the closure cap and the printed circuit board is established after releasing the first releasable connection between the closure cap and the housing or a housing part such as, for example an end cap for the housing.

The medicament delivery device may be an injection device such as, for example a fix dose or a variable dose device such as an insulin pen or an autopen. The injection device may be an autoinjector using a prefilled syringe as a medicament holder. The injection device may be a disposable device or a re-usable device or a combination thereof, a so-called semi-disposable injection device. The delivery device may also be an infusion device such as an infusion pump, a patch pump or a wearable bolus injector.

The housing may be composed of a single part or, preferably is an assembly of multiple parts that functionally and structurally behave as a housing. The housing may be an outer housing for protecting components from contamination and from mechanical damage, for example during impact. The housing may be held by the user and consequently the outer surface may have a gripping surface with 3D structures, ribs or a soft-touch (for example using 2K injection molding of a thermoplastic polymer and a thermoplastic elastomer). The housing may provide for mechanical support to other housing parts or to other mechanisms such as a dose setting mechanism, a delivery mechanism, a needle insertion mechanism, a needle retraction mechanism, a needle cover mechanism and the like. The housing also encloses and preferably supports a medicament reservoir and the delivery mechanism is configured to deliver or expel doses from the reservoir that may have been set by the dose setting mechanism.

Part of the housing may be closed by caps, for example by a pen cap on one end and by the aforementioned closure cap on the opposite end. The caps are preferably engaged with the housing by a releasable connection. The pen cap may be released by twisting and/or pulling the cap from the housing. The pen cap must be removed before expelling medicament as the injection needle must be capable for insertion into a patient as the pen cap covers the needle or the needle mounting unit. For a prefilled syringe device such as an autoinjector, the pen cap may also include a needle shield remover for simultaneous removal of the pen cap and the needle shield that covers and protects the needle before use. A pen cap may be part of a fully assembled device.

The closure cap preferably closes an end of the injection device opposite to the pen cap. Optionally, the closure cap and the pen cap are oriented perpendicular to one another. The closure cap is attached to the housing or to a housing part, for example to an end cap for the housing. The closure cap preferably defines a space between the closure cap and the housing, preferably between the closure cap and an end cap fixated to the housing. The end cap and the closure cap are preferably positioned both at the proximal end of the injection device or opposite to the end intended to be closed by the pen cap. The end cap may be the normal closing end for the injection device and the additional closure cap may be added to, or enclose an additional module for the injection device. The additional module may be an option that is added by the user or is already present in the device as manufactured. The modular approach increases the versatility of medicament delivery devices as one base unit (the housing closed by the end cap) may be combined with a plurality of modules. The additional module may be a mechanical module, an optical module, an electronic module or electro-mechanical module. The electronic module may include a switching device with an electronic switch (or electro-mechanical switch) that may be used to activate components of the electronic module. The closure cap may be part of a fully assembled device.

Preferably, the end cap closes or delimits the housing comprising mechanical components whereas the closure cap encloses a module comprising non-mechanical (for example electronic) as well as mechanical components (for example a switch). The electronic components are preferably located on a printed circuit board which may be a rigid printed circuit board or a flexible printed circuit board.

Optionally, the outside surface of the closure cap has shock absorbing properties, for example to reduce the impact forces acting on the assembled medicament delivery device during mechanical impact such as during a drop-test. The shock absorbing properties may be due to additional ribs, protrusions or 3 dimensional structures extending outwards from the closure cap. For example a plurality of ribs may be spaced apart along the longitudinal axis and extend radially outward from the closure cap. Optionally, the closure cap is made from an elastic material or the closure cap is overmolded with an elastic material such as for example a thermoplastic elastomer.

The outside surface of the closure cap may also include a clip for fixating the medicament delivery device to, for example, the clothing of a patient or a health care professional. The clip may be made from the same material as the cap (preferably a polymer) or from a different material such as a metal. The outside surface of the closure cap may also provide space for a label or QR code that is either directly printed onto the cap or the space is available for a separate label.

The first releasable connection between the closure cap and the housing, preferably between the closure cap and an end cap of the housing, may be selected from, and not limited to, a snap-fit connection, a friction fit connection, a press-fit connection, a magnetic connection, a screw type of connection, a releasable adhesive connection using for example, a pressure sensitive adhesive, or a bayonet connection.

The second connection between the closure cap and the printed circuit board may be selected from and not limited to a snap-fit connection, a friction fit connection, a press-fit connection, a magnetic connection, a screw type of connection or a bayonet connection. Optionally, the second connection is a releasable connection. The first and second connection means or connectors are configured for forming the second connection. For example by using complementary connectors or connections means.

The second connection between the closure cap and the printed circuit board is configured to be established by a first connection means or connector or fixator located on, connected to, embossed in, molded in or onto the closure cap. The first connection means is preferably located on the inner surface of the closure cap, preferably close to a rim of the cap. Alternatively, the first connection means is located on the outside surface of the closure cap. The second connection, once established utilizes the engagement between the first and second connection means. The second connection means or connector or fixator is located on, connected to, embossed in, molded in or onto, or cut-out from the printed circuit board.

Optionally, the second connection is formed using one of the two connection means or connectors only. For example, the closure cap may have the first connection means which is configured to establish a connection with the PCB having no specific second connection means.

As another option, the second connection is formed by the second connection means located on the printed circuit board engaging the closure cap having no specific first connection means.

The first and second connections means are preferably complementary to each other, for example a snap fit connection using two (first and second) snap fit connectors, or a threaded connection using two screw thread, or a protrusion engaging a complementary cut out. Further examples are presented below. The second connection is configured to be established due to a relative movement between the first and second connection means. The relative movement may be a relative axial or rotational movement or a combination thereof. For example a bayonet type of engagement requires an axial movement followed by a rotational movement.

The first and second snap fit connectors may comprise a single connector or a plurality of connectors. The plurality of first and second connectors are preferably complementary to one another and the second releasable connection may be formed whereby all connectors engage at once or whereby the connectors subsequently engage each other. The relative movement for forming the second connection may be accompanied by an acoustic and/or tactile signal. Alternatively an optical indicator or a message may be viewed, for example via a window once the second connection has been established. Optionally, the release of the first releasable connection may be accompanied by an acoustic and/or tactile signal.

A fully assembled state of the medicament delivery device preferably implies that the device is ready to use without any additional steps required by the user. Fully assembled may imply that all electronic and mechanical components including the electronic module and closure cap are assembled by the device manufacturer or the pharmaceutical company and as such ready to use. Optionally, the user may have to insert a reservoir to make the injection device ready for use. Alternatively, the reservoir and injection device may be provided as a kit of parts. In another, option a module including the closure cap is provided as a separate part and the user establishes the first releasable connection between the closure cap and the housing of the injection device before using the injection device and making the device ready to use from the kit of parts.

The second connection may be established before or simultaneous with releasing the first releasable connection.

In a less preferred option, the second connection between the closure cap and the PCB may be established before expelling medicament. A relative movement between the closure cap and the housing connects the PCB to the closure cap before medicament is expelled. The first releasable connection between the closure cap and the housing is preferably not released if the second connection is established before medicament is expelled. A disadvantage may be that the PCB is exposed to mechanical stresses before medicament delivery, for example during shelflife. The first releasable connection may be released after medicament delivery to remove the cap including the PCB from the housing. Thus releasing the first releasable connection will automatically also remove the PCB together with the closure cap from the housing of the injection device.

In a preferred option, the second connection between the closure cap and the PCB is established after expelling medicament. The second connection between the closure cap and the printed circuit board is established after medicament delivery and before or simultaneous with releasing the first releasable connection between the closure cap and the housing or a housing part such as, for example the end cap for the housing. The advantage is that during shelf-life the PCB is not stressed preventing damage to the electronic connections or electronic components on the PCB. Reducing or preventing mechanical stresses reduces the risk of delamination on the multilayered circuit boards. Securing the PCB to the closure cap has the advantage that the unit of the closure cap and the PCB with all its electronic components mounted thereon is removed as a single unit thereby providing one part that the user or health care professional can easily feed in the recycling process.

Optionally, the injection device comprises an additional securing element or a holding means which secures the printed circuit board to the housing or to an end cap of the housing or to the closure cap before expelling medicament. The securing element may establish a fit between the housing and the PCB, for example the securing element may sandwich the PCB between the housing (or end cap) and the closure cap. The additional securing means may also be called an additional holding means. The additional securing element or holding means does not expose the printed circuit board to stresses such as tensional or compressive (bending) stresses. The forces exerted onto the PCB by the securing element by the fit are minimal to prevent damage to the PCB. The additional securing element secures the PCB to the housing, or to the end cap or to the closure cap as long as the second connection has not been established yet, thus also during the medicament delivery. The additional securing element may also act as a shock absorber, for example due to the elastic properties of the securing element to improve the impact resistance of the fully assembled device including the module enclosed in or surrounded by the closure cap.

The additional securing element may be located on the closure cap and the printed circuit board may be sandwiched between the housing (or the end cap) and the closure cap using the securing element. The additional securing element may be located on one or both of the closure cap and the housing or a housing part such as the end cap. Alternatively, the securing element is part of the PCB. The additional securing element may be an elastomeric component or a spring, or a clip or an elastic arm extending from the closure cap and/or extending from the housing. The additional securing element may be released simultaneous with or after the release of the first releasable connection between the closure cap and the housing, or simultaneous with or after establishing the second connection.

The relative movement between the closure cap and the housing may be a rotational and/or axial movement and the printed circuit board is preferably rotationally locked or constrained with respect to the housing before expelling medicament such that the PCB cannot move together with the closure cap when the closure cap is moved for forming the second connection. Preferably, the PCB is rotationally fixed or locked to the housing or a housing part such as the end cap while still being axially moveable with respect to the housing or housing part. The PCB may be rotationally fixed or constrained to the housing by the securing element mentioned above or by a PCB connector temporarily connecting or constraining the PCB to the housing. The PCB preferably remains axially moveable with respect to the housing such that the PCB may be axially removed together with the closure cap from the housing once the second connection has been established and the first releasable connection has been released. The PCB connector may be a protrusion on the end cap or on the housing engaging a passage on the PCB. Optionally, a plurality of protrusions extend axially from the end cap, preferably oriented along the rotation axis and engaging a plurality of complementary passages in the PCB. The rotational movement between the closure cap and the housing may release the first connection between the closure cap and the housing and also connect the closure cap to the PCB for establishing the second connection using the first and second connection means. A relative rotation between the closure cap and the PCB is facilitated by the rotational lock between the PCB and the housing whereas the axial freedom of the PCB connector allows for removal of the closure cap together with the PCB.

Preferably the rotational movement releases the first releasable connection between the housing and the closure cap. The rotational movement preferably first forms the second connection between the PCB and the closure cap before releasing the first connection between the housing or end cap and the closure cap. Optionally, the release of the first connection occurs simultaneous to establishing the second connection. In another less preferred option the first connection is released before forming the second connection.

Optionally, the rotational movement between the closure cap and the housing before medicament delivery is prevented by a separation bolt or locking element which is operatively coupled between the closure cap and the housing. The separation bolt preferably locks the closure cap to the housing before medicament delivery and the separation bolt allows for rotational movement between the closure cap and the housing during or preferably after medicament delivery thereby allowing for the disconnection of the first releasable connection and/or establishment of the second connection. The separation bolt is operatively coupled, preferably rotationally coupled to the housing before medicament delivery and rotatable with respect to the housing during or preferably after medicament delivery.

The separation bolt may be rotationally locked to the housing by a first form fit engagement and rotationally locked to the closure cap by a second form fit engagement and wherein the separation bolt is configured to be unlocked from the housing by a delivery device enclosed in the housing.

Before delivery, the separation bolt is in the locked position and rotationally locked to the housing, thereby also rotationally locking the closure cap to the housing via the second form fit engagement. The delivery device unlocks the separation bolt, preferably by axial movement of the separation bolt and thereby releasing the first form fit engagement to the housing. The rotational movement of the closure cap relative to the housing is released and rotation of the closure cap together with the separation bolt is required for removing the closure cap from the housing. The second form fit engagement between the separation bolt and the closure cap preferably allows for axial movement between the closure cap and the separation bolt while preventing relative rotational movement. The second form fit engagement between the closure cap and the separation bolt may be released after connecting the PCB to the closure cap using the second connection.

The injection device is preferably configured to axially move the separation bolt to unlock the first form fit engagement between the separation bolt or the securing bolt and the housing.

The injection device may comprise an injection mechanism with a holding element configured to be moved in the proximal direction by an injection spring and whereby the holding element is configured to axially move the separation bolt.

The injection spring may be operationally positioned between the holding element and a plunger rod and once the injection spring is released, the holding element is capable of moving the separation bolt. Preferably the holding element directly abuts the securing bolt but alternatively an intermediate part is positioned between the holding element and the securing bolt, for example a lever arm for increasing the axial path for moving the securing bolt. The holding element is preferably moved in the proximal direction whereas the plunger rod is moved in the distal direction. The movement of the holding element towards the separation bolt and towards the end cap may be used to provide for audible feedback when starting the injection. Alternatively, the proximal movement of a signaling element that is configured to provide audible feedback at the end of the injection is used for moving the separation bolt. The signaling element is preferably tensioned by a needle cover sleeve spring and the signaling element is configured to be moved in the proximal direction once the plunger rod approaches its final distal position. The signaling element is used for producing the end of injection click. The advantage of using the signaling element for releasing the securing bolt and therewith also the first releasable connection is that the closure cap can be removed after the injection has been completed.

The injection spring of the injection device is configured to advance a plunger rod for medicament delivery.

The injection spring may advance a piston rod using the injection mechanism, for example by rotation of a driver which rotates a piston rod and which is preferably threaded engaged with the housing. The rotation of the piston rod may be due to the release of a torsional spring. Alternatively, the delivery mechanism uses a compression spring which linearly advances the plunger rod without rotation.

The second connection between the PCB and the closure cap may be a releasable connection and the printed circuit board may comprise additional release means for releasing the second connection, and may require dedicated tooling.

Preferably the release means on the PCB is separate from the second connection means. Alternatively, the second connection means may be used as release means. Preferably the PCB is rotated relative to the closure cap for releasing the second connection after the closure cap has been removed from the injection device. The release means on the PCB may be a passage, a hole or a cut-out in the PCB. A separate tool may be available for insertion into the release means and optionally for holding the closure cap. A relative rotation of the PCB with respect to the closure cap may be used for releasing the second releasable connection. The rotation is preferably in the opposite direction compared to the rotation direction for connecting the PCB to the closure cap. Removal of the PCB from the closure cap is beneficial for separation of the electronic components from non-electronic components such as the closure cap.

In an alternative option, the PCB may be released by elastically deforming the closure cap without a relative rotational movement. The first connection means on the closure cap may thereby release the PCB. The force applied for cap formation may be oriented rectangular to the position of, for example two opposite positioned first connection means.

The medicament delivery device wherein at least a part of the first connection means (or first connector or fixator) on the closure cap may be plastically deformed when establishing the second connection.

The first connection means may also be plastically deformed while simultaneously the closure cap is elastically deformed. For example a thin walled and elastically deformable closure cap may be deformed perpendicular to the longitudinal axis while simultaneously a part of the first connection means is plastically deformed.

The first and second connection means are preferably selected from a cut-out, a recess, a protrusion, a bore, a passage, a flexible arm, a ratchet, or a snap-fit connector.

As mentioned above the first and second connection means (or connectors/fixators) are complementary elements, thus a cut-out or recess is preferably combined with a protrusion or a flexible arm combined with a ratchet. The cut out or recess may have a sloped entrance for gradually establishing the connection or compressing the first and/or second connectors and gradually increasing the compressive stresses in the materials when rotating the closure cap. The cut out may be on the closure cap or on the PCB, or vice versa.

The medicament delivery device preferably is a disposable device. For a disposable device, the closure cap including the PCB/electronic module may be reused after removal from the disposable device. Optionally the delivery device itself is a reusable device and the closure cap including the PCB is removed once the battery is empty.

The closure cap may be separated from the PCB after being removed from the injection device. The closure cap as such may be reused or may be shredded. The PCB that has been separated from the closure cap may be reused or shredded. Optionally, parts of the PCB are reused, for example the battery may be reused or replaced by a new battery. Optionally, the rotational movement for establishing the second connection between the closure cap and the PCB is used for disconnecting or removing the battery from the PCB. The current inventions therefore opens a plurality of options for design for recycling.

A method for facilitating recycling of a printed circuit board with electronics contained in an injection device for expelling a medicament, the injection device comprises;
a housing, a closure cap closing the housing and connected to the housing by a first releasable connection,
a printed circuit board comprising electronic components,
the method comprises at least the following steps:
   a. establishing a second mechanical connection between the closure cap and the printed circuit board by a relative movement between the closure cap and the housing, simultaneous to, or followed by
   b. releasing the first releasable connection between the closure cap and the housing by the movement of the closure cap relative to the housing, followed by
   c. removing the closure cap together with the printed circuit board from the housing, and optionally
   d. releasing the second connection between the closure cap and the printed circuit board.

A kit of parts may be provided comprising an injection device having an end cap closing the injection mechanism, a closure cap comprising an electronic module or a closure cap separate from the electronic module.

### LEGENDS TO THE FIGURES

While the invention has been described in detail in the drawings below and foregoing general description, such description is to be considered illustrative or exemplary and not restrictive. Variations to the disclosed embodiments can be understood and effected by those skilled in the art and practicing the claimed invention, from a study of the drawings, the disclosure, and the appended claims.
- Figure 1:: Exploded view for an autoinjector,
- Figure 2a:: Longitudinal section for the autoinjector,
- Figure 2b:: Longitudinal section for the autoinjector, in a view perpendicular to Figure 2a,
- Figure 3a:: Longitudinal section of the proximal part of the autoinjector showing an electronic module in a closure cap and the locking mechanism for the release mechanism of the closure cap. Separation bolt in first position,
- Figure 3b:: Longitudinal section of the proximal part in a view perpendicular to Figure 3a,
- Figure 4a:: Longitudinal section of the proximal part of the autoinjector showing an electronic module in a closure cap and the locking mechanism for the release mechanism of the closure cap. Separation bolt in second position,
- Figure 4b:: Longitudinal section of the proximal part in a view perpendicular to Figure 4a,
- Figure 4c:: Detail of the engagement between the securing element and the locking element (or separation bolt),
- Figure 5a:: Axial mounting of the closure cap, snap fit connectors on the closure cap and on the end cap not engaged,
- Figure 5b:: Axial mounting of the closure cap, snap fit connectors on the closure cap and on the end cap partially engaged,
- Figure 5c:: Axial mounting of the closure cap, closure cap fully engaged and the locking mechanism in the locked position,
- Figure 6a:: End cap that cannot be rotated,
- Figure 6b:: End cap configured to be rotated,
- Figure 7a:: Mounting of the closure cap to the end cap shown in Figure 6a,
- Figure 7b:: Mounting of the closure cap to the end cap shown in Figure 6b,
- Figure 8a:: Assembly of end cap and closure cap before relative rotation,
- Figure 8b:: Assembly of end cap and closure cap after relative rotation,
- Figure 9a:: Cross section in a plane perpendicular to the longitudinal axis showing the snap fit connectors of the closure cap and the end cap together with a top view on the separation bolt. Separation bolt in the locked (first) position,
- Figure 9b:: Cross section in a plane perpendicular to the longitudinal axis showing the snap fit connectors of the closure cap and the end cap together with a top view on the separation bolt. Separation bolt in locked (first) position showing the engagement between the separation bolt and the closure cap,
- Figure 9c:: Cross section in a plane perpendicular to the longitudinal axis showing the snap fit connectors of the closure cap and the end cap together with a top view on the separation bolt. Separation bolt released from housing and closure cap partially twisted,
- Figure 9d:: Cross section in a plane perpendicular to the longitudinal axis showing the snap fit connectors of the closure cap and the end cap together with a top view on the separation bolt. Separation bolt released from housing and closure cap fully twisted for release of the closure cap,
- Figure 10a:: Assembly of the closure cap and the PCB before rotation of the closure cap,
- Figure 10b:: Assembly of the closure cap and the PCB before rotation of the closure cap,
- Figure 11a:: Assembly of the closure cap and the PCB after rotation of the closure cap,
- Figure 11b:: Assembly of the closure cap and the PCB after rotation of the closure cap,
- Figure 12a:: Alternative embodiment for connector on the PCB
- Figure 12b:: Alternative embodiment for connectors between the closure cap and the PCB,
- Figure 13a:: Alternative embodiment for connector on the PCB
- Figure 13b:: Alternative embodiment for connectors between the closure cap and the PCB.

### DETAILED DESCRIPTION OF THE FIGURES

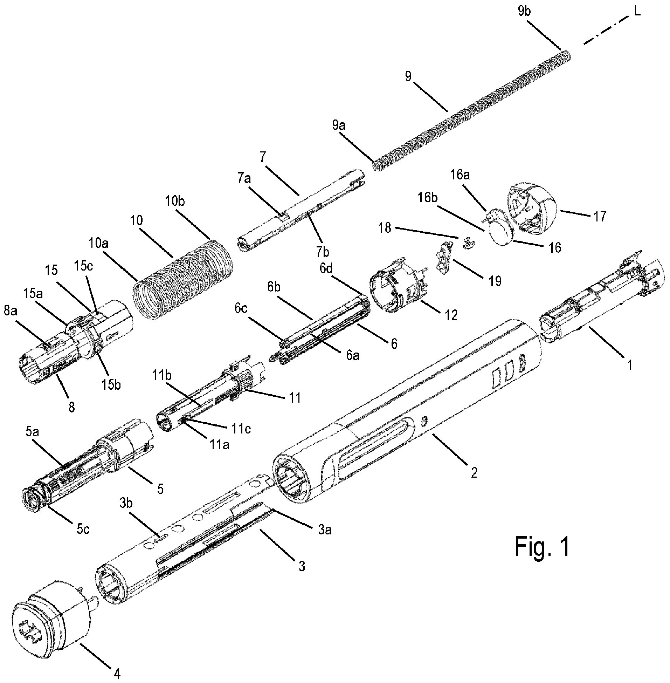

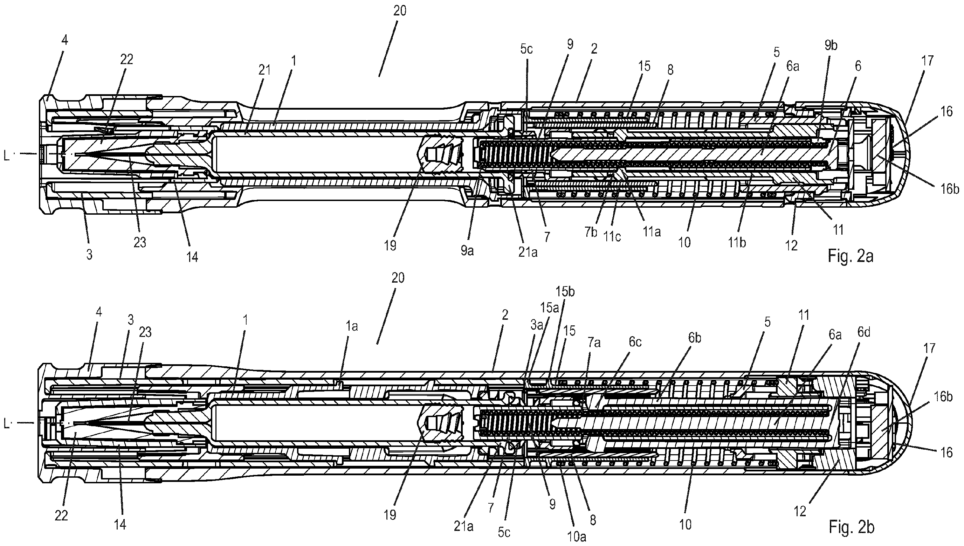

Figure 1 presents an exploded view for an autoinjector 20 and Figures 2a and 2b longitudinal sections for the autoinjector 20. An elongated sleeve shaped housing 2 can be gripped by the user during an injection and the housing defines a longitudinal axis L. An injection mechanism that is configured to be driven by a coiled compression spring is enclosed in the housing 2. A prefilled syringe 21 (Figure 2) is received in a syringe holder 1 and the syringe holder 1 is axially and rotationally secured to the housing 2 with a snap-fit connection. The autoinjector 20 as delivered to the patient is closed at the distal end by a pen cap 4 that must be removed before using the autoinjector. A needle shield 22 of the prefilled syringe 21 is coupled to the cap 4 by a needle shield remover 14 such that removal of the pen cap 4 also removes the needle shield 22 thereby exposing hollow needle 23. A needle cover sleeve 3 is axially guided by, and can be moved relative to the housing 2 along the longitudinal axis L a distance corresponding to an actuation hub. The needle cover sleeve 3 can slide in the proximal direction for actuating the injection mechanism to expel medicament from the autoinjector 20. A mechanic holder 5 is snap-fitted into and rotationally and axially secured to the housing 2. The mechanic holder 5 comprises an elastic element 5c at the distal end which abuts the proximal end 21a of the prefilled syringe 21 and biases the prefilled syringe distally into the syringe holder 1.

The injection mechanism comprises a compression spring acting as an injection spring 9. The injection spring 9 in its initial state is almost completely enclosed by a piston rod 7 and the distal end 9a of the injection spring 9 biases the piston rod 7 into the distal direction. The proximal end 9b of the injection spring abuts a holding element 6 and the holding element 6 comprises two arms 6b extending in the distal direction and one central pin 6a for guiding the injection spring 9. Each arm 6b comprises a protrusion 6c directed towards the axis L and both protrusions engage recesses 7a on the outer surface of the piston rod 7. In the initial state of the autoinjector, a switching module 8, 15 comprising switching sleeve 15 and a locking sleeve 8 prevent a deflection of the arms 6b as the arms 6b are confined within the locking sleeve 8 and therewith prevent a relative movement between the piston rod 7 and the holding element 6 thus keeping the injection spring 9 in a compressed state. The piston rod 7 is prevented from movement into the distal direction. At least the distal end 15a of the switching sleeve 15 is engaged or abuts with a proximal end 3a of the needle cover sleeve 3. The switching sleeve 15 is biased by a needle cover sleeve spring 10 into the distal direction. The distal end 10a of the needle cover sleeve spring 10 abuts a rim 15b on the switching sleeve 15 and the proximal end 10b of the needle cover sleeve spring 10 abuts a signaling element 11 and the signaling element abuts an end cap 12 that is axially and rotationally secured to the housing 2. In the initial state of the autoinjector 20, the force of the needle cover sleeve spring 10 in the proximal direction is guided to the end cap 12 of the housing 2 via the signaling element 11. In the distal direction, the needle cover sleeve spring 10 biases the needle cover sleeve 3 into the distal direction via the switching sleeve 15. For starting the injection, the needle cover sleeve 3 is pushed against the skin of the patient and moved in the proximal direction with respect to the housing 2 thereby moving the switching module 15, 8 in the proximal direction and tensioning the needle cover sleeve spring 10. In the most proximal position of the switching module 15, 8, the locking sleeve 8 may be axially engaged with the mechanic holder 5 (or alternatively to the signaling element 11 as will be discussed below) whereas the switching sleeve 15, axially guided and splined onto the locking sleeve 8, is kept by the needle cover sleeve 3 in the proximal position against the bias of the spring force provided by the needle cover sleeve spring 10.

The proximal movement of the switching module 8, 15 releases the engagement between the protrusions 6c on the holding element 6 and the recesses 7a in the piston rod 7. The spring force is released and the piston rod 7 moves into the distal direction whereas the holding element 6 can move an initial hub in the proximal direction until the holding element 6 abuts the end cap 12 of the housing 2. Optionally, an acoustic or tactile signal is generated upon abutment indicating the start of the injection. The piston rod 7 abuts a bung 19 in the prefilled syringe which is moved distally towards the outlet thereby expelling medicament through the needle 23.

The needle cover sleeve spring 10 is a metal coil spring acting as a compression spring and the proximal end 10b abuts the signaling element 11 which abuts the end cap 12 of the housing 2 before injection. The signaling element 11 comprises two longitudinally extending arms 11b each having a protrusion 11a oriented towards the longitudinal axis L. The protrusions 11a engage another recesses 7b in the piston rod 7 and the sleeves of the switching module 8, 15 ensure that the signaling element 11 is axially coupled to the piston rod 7 before and during the initial stage of the injection. The arms 11b with protrusions 11a are also confined within the locking sleeve 8 thereby preventing release of the protrusions from the recesses. During the initial stage of the injection, the piston rod 7 is advanced distally and thereby also drives the signaling element 11 in the distal direction away from the end cap 12 through the locking sleeve 8. The signaling element 11 is moved in the distal direction by a distance or tensioning hub until the protrusions 11a are released from the recesses 7b in the piston rod 7 as the arms 11b are not confined by the locking sleeve 8 anymore and can flex radially outward. The arms 11b have another protrusion 11c that is directed in the radial outward direction and those outward protrusions 11c engage the switching module 8, 15 keeping the tensioning hub of the signaling element 11. The arms 11c preferably engage the locking sleeve 8 thereby keeping the locking sleeve 8 in the proximal position (alternatively the locking sleeve engages the mechanic holder 5 as discussed above). The arms 11b can slide along the outer surface of the piston rod 7 and at the end of the injection, the piston rod has advanced such that the arms 11b of the signaling element 11 can flex radially inward again and the signaling element 11 is released from the switching module 8, 15. The signaling element 11 is biased proximally by the needle cover sleeve spring 10 and is accelerated and moved back towards the end cap 12 thereby producing an audible and/or tactile end-of-injection click.

After the medicament has been delivered, the autoinjector 20 is removed from the skin and the switching sleeve 15 together with the needle cover sleeve 3 are forwarded by the needle cover sleeve spring 10 that is at least partially decompressed. The switching sleeve 15 moves in the distal direction and is sliding along the locking sleeve 8 which has been locked before injection to the mechanic holder 5. The switching sleeve 15 may be locked in the most distal position by a flexible arm 8a of the locking sleeve 8 engaging a cut out 15c of the switching sleeve 15 thereby preventing proximal movement of the switching sleeve 15 after injection. The needle cover sleeve 3 may be locked in the most distal position by arms 1a of the syringe holder 1 that radially extend into recesses 3b of the needle cover sleeve 3 thereby preventing proximal movement of the needle cover sleeve 3 after injection.

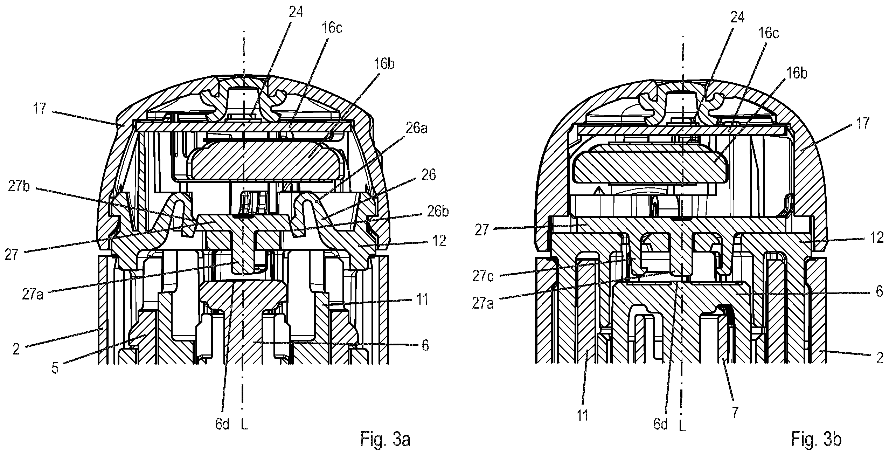

The end cap 12 is axially and rotationally fixed to the housing 2. The end cap 12 may be snap fitted, adhesively connected or welded to the housing 2. The end cap 12 can be the closure cap for the autoinjector delimiting the mechanical parts of the device in the proximal direction if there are no further modules added to the autoinjector. The end cap may provide protrusions, ribs or 3 D structures acting as shock absorbers. Preferably, the end cap 12 provides a platform or adapter for adding an additional module to the autoinjector. Preferably this is an electronic module 16 and the module is closed by a closure cap 17 engaging the end cap 12 and/or housing 2 of the autoinjector. The closure cap 17 may provide protrusions, ribs or 3 D structures on the outer surface acting as shock absorbers. Preferably, the module is a releasable module that can be released and separated from the autoinjector after injection. The electronic module 16 may comprise a sensor or switch 16a and a battery 16b and a printed circuit board 16c. The printed circuit board 16c may comprise a microprocessor, a transmitter/receiver unit, an antenna and/or a light source such as a LED 24 (Figure 3a). The light source may be used to signal the status of the device to the user, for example "ready to use" or "injection completed". The sensor 16a may be part of the PCB 16c as well and is configured to detect if the signaling element 11 is in the most proximal position. The sensor 16a may be a switch located on the PCB 16c detecting whether the signaling element 11 is in its most proximal position before injection and/or the most proximal position after injection and/or the absence of contact to the sensor during an injection. The signaling element 11 may have a protrusion extending into the proximal direction and optionally guided through passage in the end cap 12 for contacting the sensor 16a. Alternatively, the switching element indirectly contacts the sensor 16a, for example via a pivoting element or rotating element 18 that is part of or coupled to the end cap 12. The microprocessor detects the signals from the sensor 16a and is capable to transmit the data (for example time, duration, failure) of an injection to an external device using a communication module comprising, for example a transmitter and/or receiver.

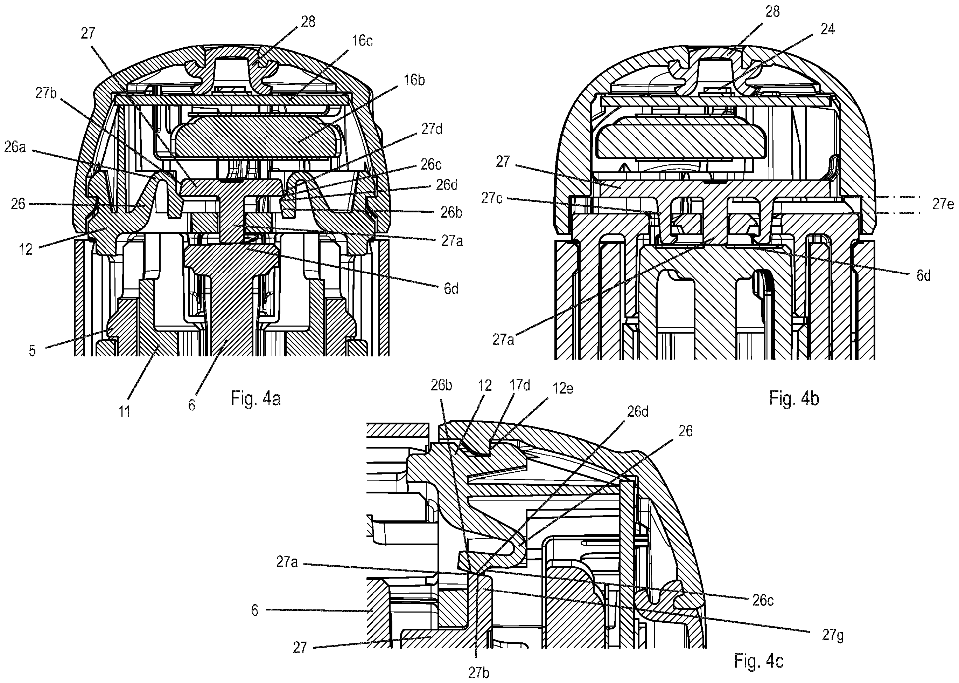

The closure cap 17 for the electronic module is preferably releasable connected to the housing 2 and/or to the end cap 12. A locking mechanism keeps the releasable connection in a locked position and the locking mechanism may be unlocked by the injection mechanism. Details are shown in Figures 3a, 3b where the locking mechanism is in the locked position, and in Figures 4a, 4b and 4c for the unlocked position.

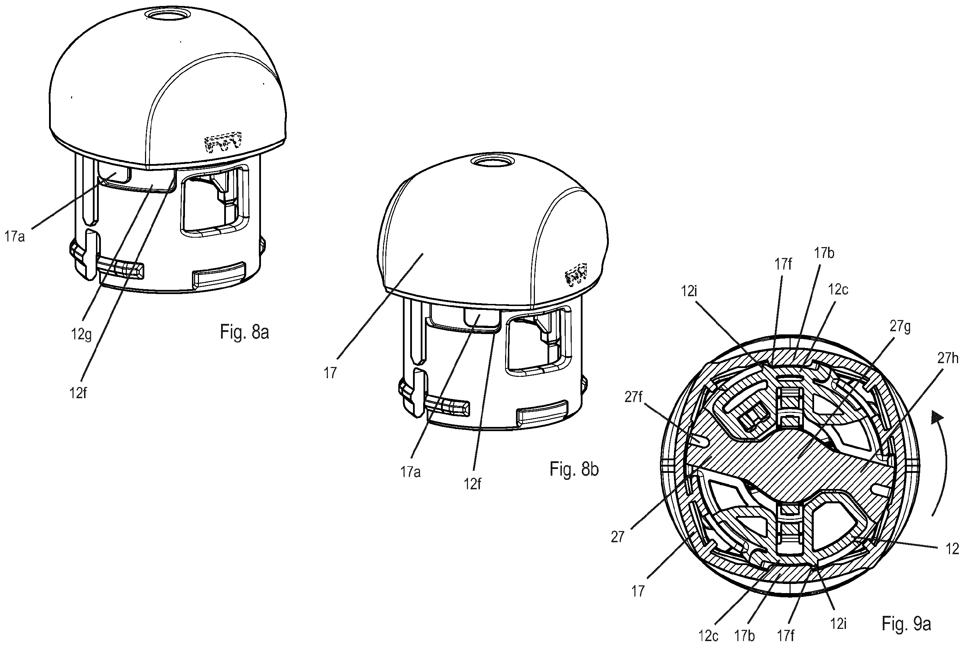

Figure 3a presents the closure cap 17 attached to the end cap 12 thereby being axially and rotationally coupled to the housing 2. The closure cap 17 encloses the printed circuit board (PCB) 16c with the battery 16b. The end cap 12 comprises a securing element 26 in the example shown in Figure 3a. Alternatively, the housing 2 comprises the securing element 26. The securing element 26 may be attached to the end cap 12 as a separate element or, preferably, the securing element 26 is integrated into the end cap 12, for example the securing element is injection molded together with the end cap 12 as a single unit. The securing element may comprise a plurality of flexible arms 26a having a U-shaped section and the end of each flexible arm 26a points towards the longitudinal axis L of the autoinjector. The flexible arms 26a may provide a bias towards the longitudinal axis when the arms are elastically deformed. More particularly once the legs of the U-shaped section are compressed towards each other. At the end of the flexible arm 26a there are two sloped surfaces 26b, 26c with a crest 26d between the two sloped surfaces (details are shown in Figure 4c). The surfaces 26b, 26c are inclined with respect to the longitudinal axis L. A locking element 27, also called separation bolt, is positioned between the electronic module and the end cap 12. The separation bolt 27 comprises wings 27h (Figure 9a) that extends radially along the end cap 12 and a protrusion 27a that extends axially along the longitudinal axis L through the end cap 12 in the distal direction (Figure 3b). Arms 27c surround the central protrusion 27a and protrude through openings in the end cap 12. Hooks at the end of the arms 27c ensure that the separation bolt 27 is axially secured to the end cap 12 while allowing for relative limited axial movement and rotation between the end cap 12 and the separation bolt 27. Figures 3a and 3b present the autoinjector before medicament delivery and the signaling element 11 abuts the end cap 12. The proximal end 6d of the holding element 6 is separated from the protrusion 27a extending from the separation bolt 27. The separation bolt 27 comprises a central disk shaped portion 27g comprising wings 27h that extend in the radial direction and the protrusion 27a extends axially from the disk shaped portion (Figures 3a, 9a). The disk 27g comprises a third sloped surface 27b at an edge of the separation bolt 27 and is being configured to engage the first sloped surface 26b on the securing element 26 before injection starts (Figures 3a, 4c). The engagement 26b, 27b biases the separation bolt 27 in the distal direction towards a first position. During start of the injection, the holding element 6 is moved in the proximal direction and the proximal end 6d of the holding element 6 abuts the separation bolt 27 such that the first surface 26b and the third surface 27b engage each other and form a gearing such that the sliding of the third surface 27b of the separation bolt 27 against the first surface 26b of the securing element 26 provide for a force that is directed radially outward thus flexing and tensioning the arm 26a. The bias of the tensioned arm with the gearing 26b, 27b tends to move the separation bolt 27 in the distal direction. As the holding element 6 continues to push on the separation bolt and surpasses a threshold, the third surface 27b passes the crest 26d on the flexible arm 26. The flexible arm 26a relaxes back to its original position as the second surface 26c engages the fourth surface 27d (or in this example point of contact) on the edge of the disk shaped portion 27g of the separation bolt 27. For details see Figure 4c. The engagement between the fourth surface 27d and the second surface 26c forms a gearing and the relaxation of the elastic energy stored in the flexible arms 26a pushes the separation bolt 27 towards the second position or the unlocked position. The separation bolt 27 can be lifted a distance indicated by gap 27e (Figure 4b). Lifting the separation bolt 27 releases a form fit engagement between the separation bolt 27 and the housing 2 or end cap 12 as will be shown below and therewith allows for rotation of the separation bolt and closure cap with respect to the housing. The closure cap 17 may be released from the housing as the locking mechanism with the locking element (or separation bolt) has been moved to the unlocked position.

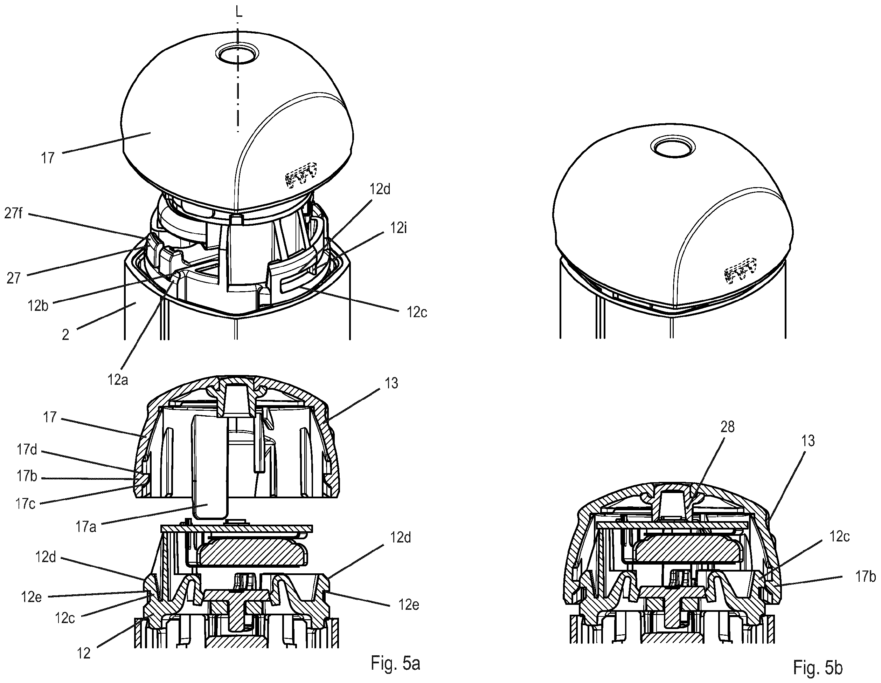

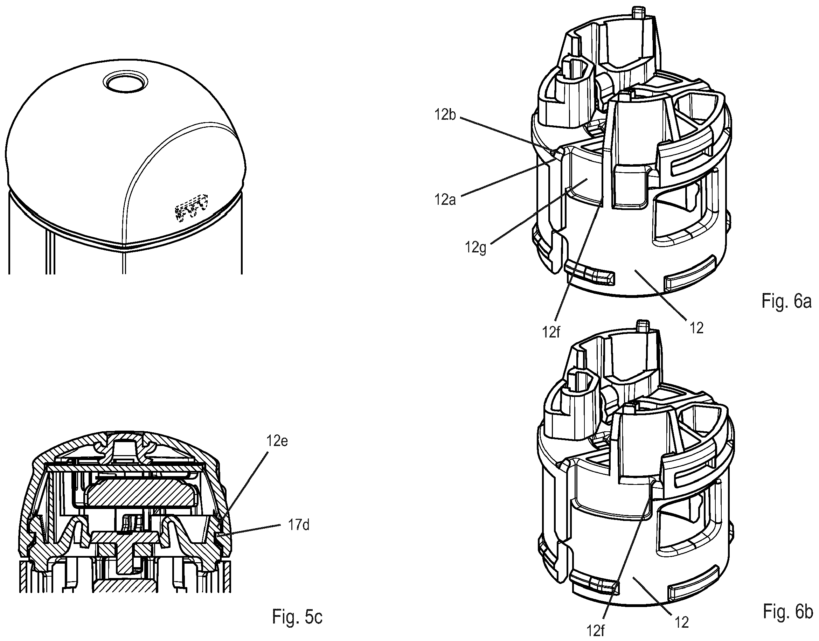

The mounting of a preferably thin walled closure cap will be described in the following (Figures 5a, 5b and 5c). The wall 13 of the closure cap that is adjacent to snap fit connectors described below has a thickness below 1 mm preferably below 0.8 mm. The closure cap 17 is moved along the longitudinal axis L towards the end cap 12 or the housing 2 (Figure 5a). The end cap 12 comprises at least one snap fit connector 12c comprising a first sloped surface 12d which is tilted with respect to the longitudinal axis L and a first stopping surface 12e oriented essentially perpendicular to the longitudinal axis L. The closure cap 17 comprises at least one snap fit connector 17b having a second sloped surface 17c complementary to the first sloped surface 12d on the snap fit connector 12c on the end cap 12. The snap fit connector 17b comprises a second stopping surface 17d configured to engage the first stopping surface 12e on the end cap 12. The first and second sloped surfaces 12d, 17c slide over each other as the closure cap 17 approaches the end cap 12 and the engagement between the sloped surfaces 12d, 17c results in a force directed radially outward which elastically deforms the preferably thin walled closure cap 17 (Figure 5b). The stopping surfaces 12e, 17d engage each other as the closure cap has reached its final axial position and the closure cap can elastically relax back to its initial position (Figure 5c). The engagement of the stopping surfaces 12e, 17d prevent axial rearward movement of the closure cap 17 and thus unintended release of the closure cap from the end cap before injection.

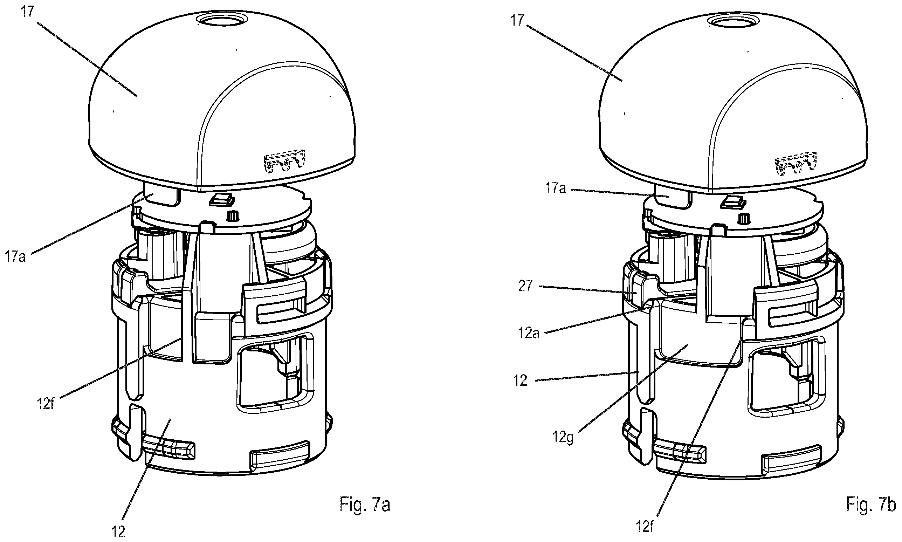

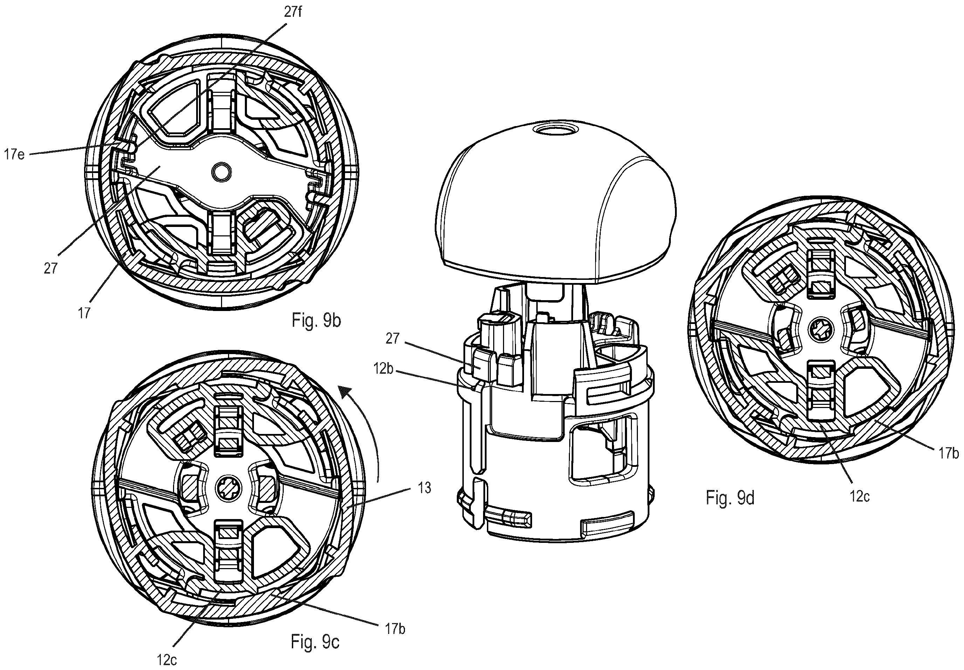

Rotation of the closure cap 17 with respect to the housing or end cap 12 is prevented before releasing the locking mechanism by the injection mechanism. The closure cap 12 is rotationally locked to the separation bolt 27 as the wings 27h comprise a groove 27f that extends along the axis L and the grooves 27f engage a longitudinal key 17e on the closure cap (Figures 5a, 9a, 9b). A radial stop 12a on the end cap 12 abuts an edge of the wings 27h (Figure 7b) thereby preventing rotation of the separation bolt 27 and thus also rotation of the closure cap 17 via the key/groove engagement 27f, 17e (Figure 9b). Once the separation bolt 27 has been lifted by the injection mechanism a distance 27e exceeding step 12b, then the separation bolt 27 and therewith also the closure cap 17 may rotate (Figure 9d).

The maximum rotation of the closure cap may be limited by a radial stop 12f engaging a protrusion 17a axially extending from the closure cap 17. The end cap 12 may form a guide slot 12g for the protrusion. The position of the radial stop 12f defines the maximum angle of rotation. In Figure 6a, the radial stop 12f is positioned such that the protrusion 17a snuggly fits in thereby allowing no or only limited rotation of the closure cap (Figure 7a). In Figure 6b, the protrusion may rotate a certain angle until the protrusion 17a abuts radial stop 12f (Figure 7b). Figures 6a and 6b show two different embodiments for the end cap 12 with a different position of the radial stop 12f. The end cap is injection molded and the two versions can be made from one modular tooling by simply removing or adding one part to the mold.

The closure cap 17 may be released from the housing 2 and/or end cap 12 by relative rotation between the closure cap 17 and the housing (preferably after the injection mechanism has released the locking mechanism for the separation bolt). The assembly of a closure cap 17 that cannot rotate with respect to the end cap 12 is presented in Figure 7a. The protrusion 17a fits into and abuts the radial stop 12f preventing relative rotation between the two caps. Figures 7b, 8a and 8b represent an assembly of the closure cap 17 that may rotate with respect to the end cap once the locking mechanism has been released. The protrusion 17a may move in the guide slot 12g until the protrusion abuts the radial stop 12f (Figure 8b). The rotation of the closure cap 17 is transferred into a rotation of the separation bolt or locking element 27 via the key/groove interaction 27f, 17e (Figure 9b).

Rotation of the preferably thin walled closure cap 17 may elastically deform the closure cap. The snap fit connector 12c on the end cap 12 comprises a third sloped surface 12i that is oriented in the circumferential direction (Figures 5a, 9a). The third sloped surface 12i may be on the same snap fit connector 12c having the first sloped surface 12d that is oriented in the axial direction as shown in Figure 5a. Alternatively the third sloped surface 12i is on a separate snap fit connector 12c. The third sloped surface 12i and the first sloped surface 12d are essentially oriented perpendicular to one another. The snap fit connector 17b on the closure cap comprises a fourth surface 17f oriented in the circumferential direction and complementary to the third sloped surface 12i on the snap fit connector 12c on the end cap 12 (Figure 9a). Rotation of the closure cap 17 with respect to the housing brings the third and fourth sloped surfaces 12i, 17f in engagement such that the relative movement between the sloped surfaces provides for a force in the radial direction thereby elastically deforming the closure cap 17 (Figure 9c). Once the closure cap has been fully rotated, optionally until the protrusion 17a of the closure cap abuts the radial stop 12f on the end cap, then the snap fit connectors 12c, 17b are disconnected (Figure 9d) and the closure cap 17 relaxes back to its original shape. The closure cap 17 may be axially removed from the injection device as this axial movement is allowed by the key/groove interaction 27f, 17e.

The stiffness of the snap fit connector 12c on the end cap 12 is greater than the stiffness of the closure cap 17 and/or the stiffness of the snap fit connector 17b on the closure cap. The stiffness may be tuned by the geometrical dimension, for example using a thin walled closure cap 17 with respect to snap fit connectors on the end cap having a greater thickness for example at least twice, preferably triple the thickness of the closure cap. Alternatively, the snap fit connector 12c on the end cap is made from a material having a greater stiffness, for example by using a technical material such as a glass fibre reinforced plastic. The closure cap may be made from a material having a relatively low stiffness for example (expressed in the young's modulus) below 3 GPa, preferably below 2 GPa. The closure cap may be made for example from polypropylene, polyethylene, polycarbonate, ABS, polystyrene, a polyester such as polyethyleneterephthalate, a cycloolefinic polymer (COC or COP) or a polyamide. The closure cap may also be made from a blend of polymers such as polycarbonate/ABS or PPO/Polystyrene. Preferably, the closure cap 17 is thin walled and/ or made from a material having a relatively low stiffness as this saves material and costs.

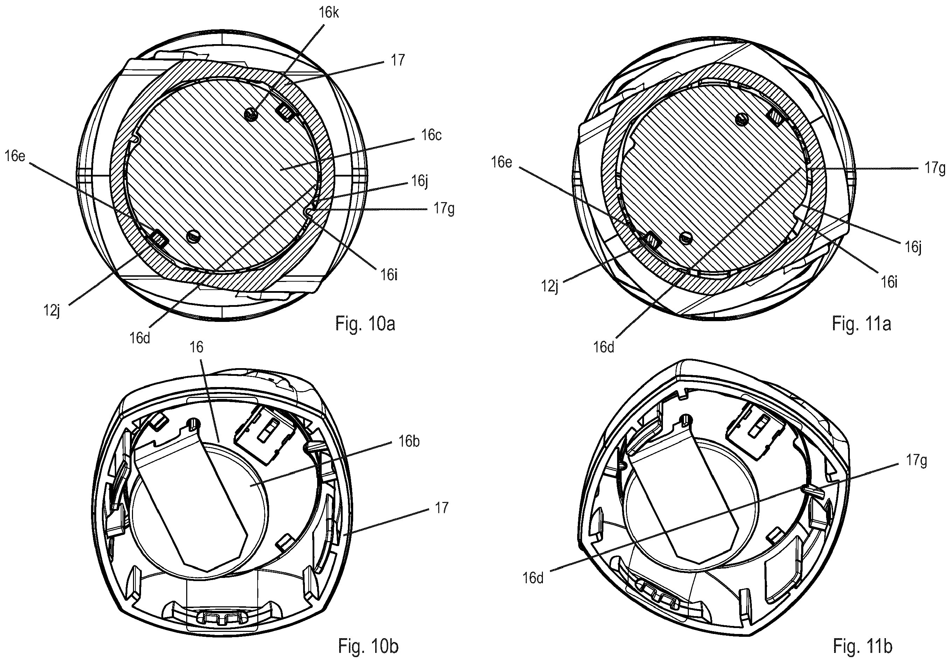

The fixation of the electronic module 16, preferably the fixation of the printed circuit board 16c is shown in Figures 10 to 13. The injection device is preferably fully assembled meaning that the closure cap 17 including the electronic module 16 is attached to the housing 2. Preferably, the manufacturer of the device has attached the electronic module to the injection device. Optionally, the injection pen closed by the end cap 12 is delivered as a kit of parts together with the closure cap 17 and the electronic module 16. The user or health care professional attaches the closure cap 17 with the electronic module 16 to the housing 2 or end cap 12 just prior use thereby obtaining the fully assembled injection device.

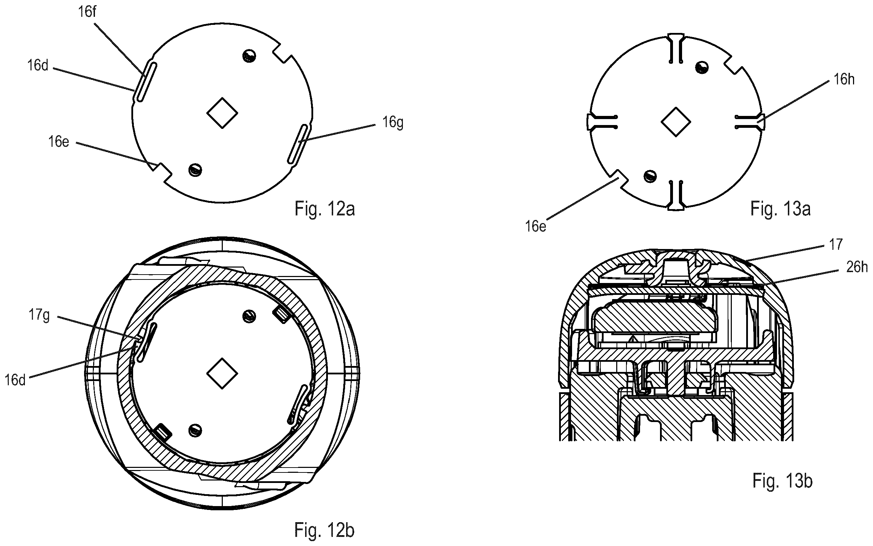

The PCB may be releasable connected to the closure cap once the closure cap 17 can be rotated and the connection between the closure cap and the housing has been released. The PCB 16c may be fixated to the closure cap 17 simultaneous with the release of the closure cap from the end cap, or, more preferable, the PCB 16c is fixated before the release of the closure cap and preferably using the same rotational movement. The PCB 16c is preferably rotationally fixated to the end cap 12 by cut outs 16e engaging protrusions 12j on the end cap 12 that extend in the proximal direction such that the PCB 16c cannot co-rotate with the closure cap 17 (Figure 10a). The engagement 12j, 16e allows for relative axial movement between the PCB 16c and the end cap 12 (Figure 10a). The closure cap 17 comprises a first connector 17g, preferably a protrusion that extends radially inward which fits into a cut out 16i in the PCB 16c before rotation of the closure cap 17. The PCB may have a second connector or connection means that is angular displaced from the cut out 16i. The cut out 16i may have a sloped section 16j. Rotation of the closure cap 17 in the counterclockwise direction rotates the closure cap 17 relative to the PCB 16c. The first connector or protrusion 17g is rotated relative to the PCB and optionally may be first compressed by the sloped section 16j on the PCB. The first connector 17g may finally engage the second connector 16d on the PCB 16c (Figure 11a). In this example, the second connector 16c represents the normal edge of the PCB without any modifications. Alternatively, the second connector 16c may be formed by another cut-out in the edge of the PCB. During rotation of the closure cap, the first connector 17g is elastically and/or plastically deformed to establish a connection between the closure cap 17 and the PCB 16c. The PCB is based on a rigid board and preferably does not deform during rotation of the closure cap 17. The closure cap 17 may be removed from the injection device together with the electronic module 16 and used for recycling purposes (Figure 11b). The PCB 16c may have additional holes 16k (also called release means) that may be used during recycling for rotating back (in the clockwise direction) the PCB 16c and releasing the PCB from the closure cap 17. Alternatively, the cut outs 16e may be used for releasing the PCB 16c from the closure cap 17. Alternatively, the elastic deformation is provided by the PCB (Figures 12 and 13). The PCB 16c may have slots 16g leaving thin sections 16f of the PCB such that radial forces may elastically deform the PCB 16c during rotation of the closure cap 17 (Figure 12b). Alternatively, the PCB comprises arms 16 that protrude from the PCB 16c and that can flex such that the end of the arms may engage an internal surface of the closure cap (Figures 13a, 13b).Alternatively, the closure cap is elastically deformed for releasing the PCB (thus without using a relative rotational movement between the closure cap and the PCB).

The closure cap 17 may have an additional holding element or securing element 28 which sandwiches the electronic module 16, preferably the PCB 16c between the closure cap 17 and the housing 2 or the end cap 12 (Figures 4a, 4b). The securing element 28 may be an elastomeric member or a flexible arm or a combination thereof providing an interference fit for the PCB without deforming or stressing the PCB. The securing element presented in Figures 4a, 4b is an elastomeric element fitted into the dome of the closure cap and having a rim or wings extending distally that can flex and abut the proximal surface of the PCB. Before use, the PCB is fixated to the end cap 12 or housing 2 and is not loose within the space formed by the closure cap 17. The absence of mechanical stresses improves the reliability of the electronic module. After expelling medicament, the second releasable connection is formed between the closure cap 17 and the PCB and the connection may stress the PCB which may not disadvantageous as the electronic module has already been activated and used. The second releasable connection is formed for recycling purposes whereas the interference fit provided by the securing element before expelling medicament ensures that the module cannot move or vibrate and improves the impact resistance without jeopardizing the reliability of the PCB and its components.

**PART ANNOTATION**

| | | | |
|---|---|---|---|
| 1 | Syringe holder | 12f | Radial stop |
| 1a | Arms | 12g | Guide slot |
| 2 | Housing | 12i | Third sloped surface |
| 3 | Needle cover sleeve | 12j | Protrusion |
| 3a | Proximal end | 13 | Wall |
| 3b | Recess | 14 | Needle shield remover |
| 4 | Pen cap | 15 | Switching sleeve |
| 5 | Mechanic holder | 15a | Distal end |
| 5c | Holding spring | 15b | Rim |
| 6 | Holding element | 15c | Cut out |
| 6a | Central pin, guide pin | 16 | Electronic module |
| 6b | Arms | 16a | Sensor/switch |
| 6c | Protrusion | 16b | Battery |
| 6d | Proximal end | 16c | PCB |
| 7 | Piston rod | 16d | Second connector |
| 7a | Recess | 16e | Cut out |
| 7b | Recess | 16f | Thin section |
| 8 | Locking sleeve | 16g | Slot |
| 8a | Flexible arm | 16h | Arm |
| 9 | Injection spring | 16i | Cut out |
| 9a | Distal end | 16j | Sloped section |
| 9b | Proximal end | 16k | Hole / passage |
| 10 | Needle cover sleeve spring | 17 | Closure cap |
| 10a | Distal end | 17a | Protrusion, guide pin |
| 10b | Proximal end | 17b | Snap fit connector |
| 11 | Signaling element | 17c | Second sloped surface |
| 11a | Protrusion | 17d | Second stopping surface |
| 11b | Arms | 17e | Key |
| 11c | Protrusion | 17f | Fourth sloped surface |
| 12 | End cap | 17g | First connection means |
| 12a | Radial stop | 18 | Pivoting element |
| 12b | Step | 19 | Bung |
| 12c | Snap fit connector | 20 | Prefilled syringe |
| 12d | First sloped surface | 21a | Proximal end |
| 12e | First stopping surface | 21 | Needle shield |
| 22 | Hollow needle | 27c | Arms, hooks |
| 23 | LED | 27d | Fourth surface |
| 24 | Passage end cap | 27e | Gap |
| 25 | Securing element | 27f | Groove |
| 26a | Flexible arm | 27g | Centre portion |
| 26b | First sloped surface | 27h | Wings |
| 26c | Second sloped surface | 27 | Securing element |
| 26d | Crest | L | Longitudinal axis |
| 26 | Locking element, separation bolt | 8,15 | Switching module |
| 27a | Protrusion | | |
| 27b | Third sloped surface | | |

## Claims

1. A medicament delivery device for expelling fluid medicament comprising:
a housing (2),
a closure cap (17) closing the housing (2) and connected to the housing by a first releasable connection, and
a printed circuit board (16c) comprising electronic elements, the printed circuit board (16c) being located in the closure cap (17),
a first connection means (17g) located on the closure cap (17) and a second connection means (16d) located on the printed circuit board (16c),
wherein a second connection is configured to be established between the closure cap (17) and the printed circuit board (16c) by engaging the first and second connection means (17g, 16d) through a relative movement between the closure cap (17) and the housing (2) in a fully assembled state of the medicament delivery device.

2. The medicament delivery device according to claim 1 wherein the second connection is established before, or simultaneous with, releasing the first releasable connection.

3. The medicament delivery device according to claims 1 or 2 wherein the second releasable connection is established after expelling medicament.

4. The medicament delivery device according to claim 3 wherein additional securing element (28) which secures the printed circuit board (16c) before expelling medicament by an interference fit.

5. The medicament delivery device according to claim 4 wherein the additional securing element (28) is located on the closure cap (17) and wherein the printed circuit board (16c) is sandwiched between the housing (2) and the closure cap (17).

6. The medicament delivery device according to claims 3 to 5 wherein the relative movement between the closure cap (17) and the housing (2) is a rotational movement and wherein the printed circuit board (16c) is rotationally locked with respect to the housing (2).

7. The medicament delivery device according to claim 6 wherein the rotational movement releases the first releasable connection between the housing (2) and the closure cap (17).

8. The medicament delivery device according to claim 7 wherein the rotational movement between the closure cap (17) and the housing (2) before medicament delivery is prevented by a separation bolt (27) operatively coupled between the closure cap (17) and the housing (2).

9. The medicament delivery device according to claim 8 wherein the separation bolt (27) is rotationally locked to the housing (2) by a first form fit engagement and rotationally locked to the closure cap (17) by a second form fit engagement and wherein the separation bolt (27) is configured to be unlocked from the housing (2) by an injection mechanism enclosed in the housing (2).

10. The medicament delivery device according to claim 9 wherein the injection mechanism is configured to axially move the separation bolt (27) to unlock the first form fit engagement between the separation bolt (27) and the housing (2).

11. The medicament delivery device according to claim 10 wherein the injection mechanism comprises a holding element (6) which is configured to be moved in the proximal direction by an injection spring (9) and whereby the holding element (6) is configured to axially move the separation bolt (27).

12. The medicament delivery device according to any of the previous claims wherein at least a part of the first connection means (17g) on the closure cap (17) is plastically deformed when establishing the second releasable connection.

13. The medicament delivery device according to any of the previous claims wherein the first and second connection means (17g, 16d) are selected from a cut-out, a recess, a protrusion, a bore, a passage, a flexible arm, or a snap-fit connector.

14. A method for facilitating recycling of a printed circuit board with electronic components contained in a medicament delivery device for expelling a fluid medicament, the delivery device comprises:
a housing,
a closure cap closing the housing and connected to the housing by a first releasable connection,
a printed circuit board comprising electronic components, the printed circuit board being located in the closure cap,
the method comprises at least the following steps:
a. establishing a second mechanical connection between the closure cap and the printed circuit board by a movement of the closure cap relative to the housing, simultaneous to, or followed by,
b. releasing the first releasable connection between the closure cap and the housing by the movement of the closure cap relative to the housing,
c. removing the closure cap together with the printed circuit board from the housing.

15. The method for facilitating recycling according to claim 14 further comprising the following step:
d. releasing the second mechanical connection between the closure cap and the printed circuit board.
